# EUROPEAN PATENT APPLICATION

(11) **EP 3 290 081 A1**
(43) Date of publication of application: **07.03.2018**
(21) Application number: 16202110.9
(22) Date of filing: 05.12.2016
(51) Int. Cl.: A61N 1/05, A61N 1/375

(54) **IMPLANTABLE LEADLESS PACEMAKER WITH CAPACITIVE ELECTRODE**

(30) Priority: 06.09.2016 US 201662383606 P
(71) Applicant: BIOTRONIK SE & Co. KG, 12359 Berlin (DE)
(72) Inventor: Baru, Marcelo, Tualatin, OR 97062 (US)
(74) Representative: Galander, Marcus

(57) **Abstract**

An implantable leadless cardiac pacemaker with at least one capacitive electrode and an embedded anti-parallel Schottky junction. The leadless pacemaker can include a bonded semiconductor die, depositing a metal layer, an oxide layer, an insulating layer, and/or a conductive layer over at least a portion of the leadless pacemaker case. Some embodiments can include attaching an n-type silicon ("Si") die to a portion of the pacemaker case. With the various configurations, a Schottky junction can be formed with the n-type Si die, which can be used to limit current flow in one direction and provide protection from external defibrillation pulses.

## Description

Embodiments of the disclosed invention generally relate to a pacemaker system using a capacitive electrode and, in particular, a capacitorless leadless pacemaker system that may include an embedded anti-parallel Schottky junction for oxide reverse voltage protection in the presence of external defibrillation pulses.

Leadless cardiac pacemakers can be small form-factor devices that may be anchored directly to interior walls of a heart. Generally, leadless cardiac pacemakers are powered by a primary battery. Due to placement within the body and the nature of use, there is an impetus to miniaturize the electronic module and other components of the leadless cardiac pacemaker to accommodate efforts in reducing implant size and increase battery capacity as much as possible given the available maximum volume.

Traditional pacemaker systems consist of pacemaker housings containing electronic modules with electrical connection to noble-metal electrodes (*e.g*., fractal iridium) via direct current ("DC") blocking capacitors arranged in electrical series with the electrodes. For example, conventional leadless pacemaker systems may include a single DC blocking capacitor as these systems are single-chamber pacemakers. This arrangement is typically used to enable compliance with DC leakage requirements (*i.e.,* inhibit leakage of DC current from the electronic module) and to provide protection against single-faults in the electronic module. DC blocking capacitors typically have a capacitance of several micro-Farads ("µF"), thus it is common for them to be large discrete components so as to handle requisite operational parameters, detracting from the form-factor aspects of the pacemaker and hampering miniaturization efforts. In present leadless pacemaker designs, there is at least one DC blocking capacitor provided as these are single chamber devices. Further, use of discrete components can result in an increase of a number of component parts used to fabricate the pacemaker system, a consumption of otherwise useful space within the pacemaker housing, and/or an increasing the volume of space occupied by the pacemaker system, all of which can increase cost and complexity.

Capacitive electrodes tend to be ideal electrodes for pacemaker systems because they can operate without any faradaic reactions at the electrode-tissue interface. A practical material used for generating capacitive electrodes can be anodized tantalum ("anodized Ta"), which may be partially due to its appropriate charge storage capacity and its resistant to corrosion when placed within a saline environment. Further, Ta-based materials are used extensively as biological implant materials. A typical anodized Ta material can be tantalum pentoxide ("Ta₂O₅"). One fact to consider though is that Ta₂O₅ based capacitive electrodes are polarizable. Because cathodic stimulation is generally physiologically preferred with the use of pacemaker systems and because Ta₂O₅ based capacitive electrodes are polarizable, Ta₂O₅ based capacitive electrodes may have to be pre-biased to a positive voltage when they are used as the stimulation electrode of the pacemaker.

As an alternative, the capacitive electrode can be used as the return electrode in the stimulation to avoid the need for pre-biasing. Yet, this may require pre-charging the polarizable capacitor through tissue for stimulation, as disclosed in U.S. Patent No. 5,312,439. This method may not be feasible for cardiac pacing, as sensing of intrinsic cardiac events occurs through the same electrodes.

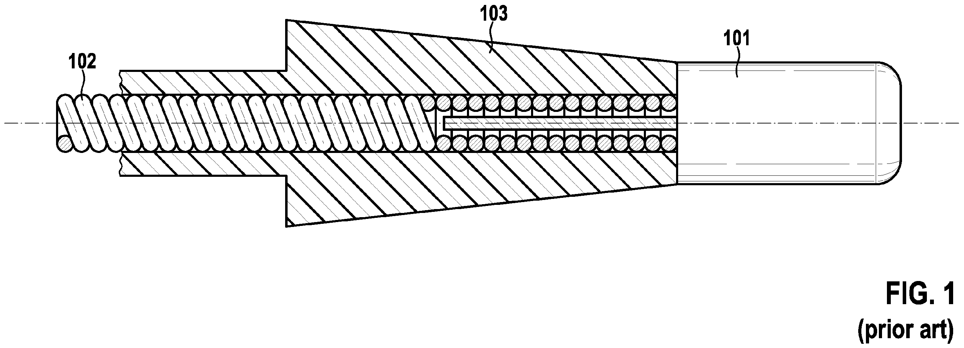

Traditionally, pacemakers with leads based on capacitive electrodes for implantable pacemakers have consisted of electrodes including a metal tip coated by a thin dielectric layer of an electrochemically-deposited oxide (*e.g.,* Ta oxide) 101. A helical Cobalt-Chromium-Nickel Alloy wire 102 may be encapsulated in a silicone elastomer medical grade tubing 103 to form a connection to a pacemaker pulse generator. (*See* FIG. 1).

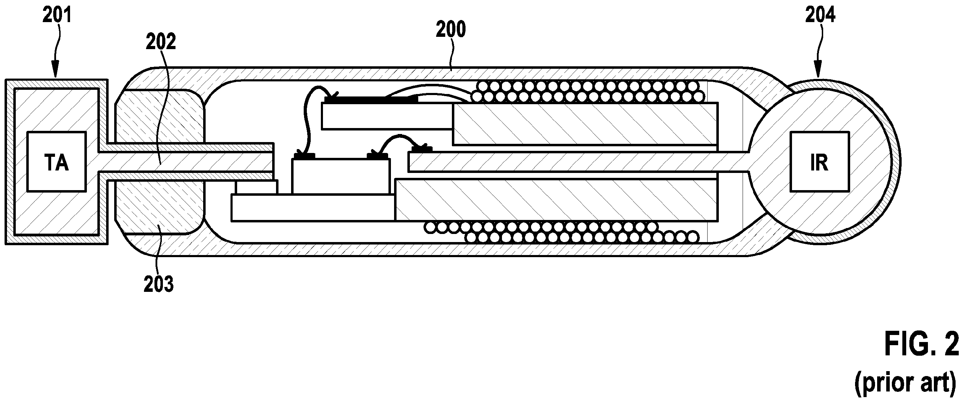

Other prior art capacitive electrode lead systems or implantable stimulators may include a glass-encased radio frequency powered micro-stimulator with a cylindrical-shaped Ta oxide coated open cell capacitive electrode 201 at an anodal pulse end attached via a Ta stem 202, which may be hermetically sealed through a glass bead 203 into an inside of a glass container 200. (*See* FIG. 2). In such systems, the return electrode may be an iridium ("lr") electrode 204. Such a system is disclosed in U.S. Patent No. 5,312,439.

Other approaches, such as disclosed in U.S. Patent No. 6,035,237, may include an implantable stimulator configured to avoid use of conventional coupling capacitors in an output stage of the pacemaker system. With such a configuration, a pair of electrodes may be made of a material that allows them to function as a capacitor. Further, sintered Ta may be placed on the pair of electrodes of the stimulator, which can cause them to act as a single bipolar (non-polarizable) capacitor.

Further approaches, such as disclosed in U.S. Patent No. 7,831,309, may include intraocular and periocular implantation stimulation devices that utilize Ta electrodes covered by Ta₂O₅ to AC-couple the stimulation circuit. Moreover, a stimulation electrode with an electrically conducting base body may be used, which can include use of Ta layer and a porous Ta oxide layer, as per U.S. Patent No. 8,594,808. A metallic protective layer may then be used to cover the porous Ta oxide layer to prevent hydrogen embrittlement.

The prior art pacemaker systems including leads based on capacitive electrodes use the oxide layer as an insulator to prevent direct current from flowing between the electrodes and through tissue when the devices are implanted. However, an important limitation of capacitive electrodes in prior art devices is that no provision is made for protection against external defibrillation pulses. It is not uncommon for a patient having a pacemaker implant to be subjected to external defibrillation therapy, and when done so this oxide layer can be irreparably damaged, possibly resulting in a hazardous condition for the patient. While conventional pacemakers and implantable stimulator based on capacitive electrodes in prior art may have built-in protections as part of the electronic module to assist with preventing damage to the device when subjected to defibrillation pulses, none provide protection for the oxide layer and/or provide protection to the pacemaker system when the oxide layer becomes damaged. For example, a typical form of protection used by conventional pacemaker systems is the use of clamping Zener diodes at the front-end circuitry, which may permit a temporary reverse voltage build-up across the electrodes in the range of tens of volts. However, when a polarizable capacitive electrode is used, this reverse voltage may damage the oxide layer beyond self-healing, resulting in a catastrophic failure of the electrode as a DC blocking element. Further, using polarizable capacitive electrodes in both the stimulating electrode and the return electrode, as disclosed in U.S. Patent No. 6,035,237, while utilizing such a protection scheme, would still result in the same problem. This is because one of the polarizable capacitive electrodes would still experience a reverse voltage when being subjected to external defibrillation pulses. Thus, the prior art fails to provide adequate protection against such external defibrillation pulses.

The present invention is directed toward overcoming one or more of the above-identified problems.

It is an object of the present invention to remove the need for such DC blocking capacitors on the electronic module by using at least a capacitive electrode based on a dielectric.

Embodiments disclosed herein are directed toward a pacemaker system with at least one capacitive electrode and a method of producing the same. The pacemaker system can be an implantable cardiac pacemaker system, and further be a leadless implantable cardiac pacemaker system. The leadless pacemaker system can include a pacemaker case with a pacing electrode (*i.e.,* stimulating electrode) and a return electrode, wherein an arrangement of a plurality of layers, bonded die, and coatings deposited on at least some portions of the pacemaker system may be used to provide various forms of protection against external defibrillation pulses. In some embodiments, the leadless pacemaker system can use the capacitive electrode as a return electrode. Further embodiments can include use of an embedded or attached anti-parallel Schottky junction.

The pacemaker system can include depositing a metal layer over at least a portion of the pacemaker case, and may further include growing an oxide layer on a selective area of the pacemaker case, for example on at least a portion of the deposited metal layer. Any one or both of the metal layer and the oxide layer can be partially electrically isolated by an application of an insulating coating layer. A conductive layer may then be deposited over at least a portion of the oxide layer and/or the insulating coating layer. Further embodiments can include attaching a semiconductor die, for example a n-type silicon ("Si") die to at least a portion of the pacemaker system, forming an ohmic contact with the portion connected thereto, with the conductive layer described above covering the unattached die face as well. With the various layer arrangements and/or use of the die, the pacemaker system can be made to form a Schottky junction with the die, which can be used to limit current flow in one direction and provide further protection from external defibrillation pulses. Further, an equivalent capacitance of the capacitive electrode can be made to be greater than an equivalent capacitance of the insulating coating layer portion of the leadless pacemaker case.

In at least one embodiment, an anti-parallel Schottky junction can be built into an electrode assembly of the pacemaker system to provide reverse voltage protection in the presence of external defibrillation pulses by accommodating polarizable characteristics of the capacitive electrode. In further embodiments, the conductive layer may be applied to at least a portion of the electrode assembly and further act as a cathode of the capacitive return electrode (anode of the anti-parallel Schottky junction). This conductive layer can also provide added protection to the oxide layer underlying it, which may include protection against handling (*e.g*., scratching and abrasion) and other environmental effects.

The leadless pacemaker system can be structured to obviate use of large discrete components (*e.g*., series DC blocking capacitor), and in doing so reduce the number of parts used to fabricate the pacemaker (*e.g*., reduce component count), reduce the volume of space utilized within a housing of the pacemaker, and/or the volume of space the pacemaker system itself occupies. Unlike conventional pacemaker systems, the disclosed pacemaker system can meet DC leakage requirements without any type of discrete component placed in electrical series with the pacemaker pacing circuit. Further, the pacemaker system can be configured without any type of additional discrete component (*e.g.,* DC blocking capacitor) for direct current blocking by using at least a capacitive electrode based on a dielectric. Additionally, embodiments of the present invention can provide for a leadless pacemaker system with built-in series capacitor, and one that achieves oxide reverse voltage protection without voltage pre-biasing. This may include providing oxide reverse voltage protection in the presence of external defibrillation pulses.

In an exemplary embodiment, a pacemaker can include a capacitive electrode and a metal-based second electrode, where at least an electrode may be connected to the pacemaker case. The pacemaker can be an implantable cardiac pacemaker, and further be a leadless implantable cardiac pacemaker. Further, the capacitive electrode can be a capacitive return electrode, and the second electrode may be a metal-based stimulating electrode. A metal layer can be deposited on at least a portion of the pacemaker case. An oxide layer can also be deposited on at least a portion of the pacemaker case to form the capacitive electrode. A Schottky junction may be bonded to the pacemaker case such that one terminal is electrically-connected to the capacitive electrode. Further, an insulating layer may be used to electrically isolate most of the portion of the pacemaker case without the oxide layer. A conductive layer may be deposited on at least a portion of the oxide layer and/or the insulating coating layer, and the unattached die face. In addition, the Schottky junction can prevent irreparable damage to at least the oxide layer when the pacemaker is subjected to external defibrillation pulses without voltage pre-biasing the capacitive electrode. Further, the capacitance of the capacitive electrode can be greater than a capacitance of the insulating coating layer portion of the pacemaker case. In some embodiments, the conductive layer serves as a cathode of the capacitive electrode. Further embodiments may include Ta₂O₅ layer as the oxide layer. In at least one embodiment, the pacemaker can include a battery can containing an assemblage of battery chemistry and battery components, wherein the anode of the capacitive electrode may be connected to a negative terminal of the battery can. At least a portion of the battery can may serve as the pacemaker case.

In another exemplary embodiment, a pacemaker can include a pacemaker case, a capacitive electrode and a metal-based second electrode, a tantalum ("Ta") layer deposited on at least a portion of the pacemaker case, a tantalum pentoxide ("Ta₂O₅") layer deposited on at least a portion of the Ta layer, an insulating coating layer partially applied to the Ta layer and/or at least a portion of the Ta₂O₅ layer, and a conductive layer deposited on at least a portion of the Ta₂O₅ layer and/or the insulating coating layer. Further, a Schottky junction may be bonded to the pacemaker case such that one terminal is electrically-connected to the capacitive return electrode. Alternatively or additionally, a Schottky junction can be formed on at least a portion of the pacemaker case and can be in electrical connection with the capacitive return electrode. The Schottky junction may provide oxide reverse voltage protection to the pacemaker when the pacemaker is subjected to external defibrillation pulses. The Ta₂O₅ layer can be structured to have a porous outer layer and a dense inner layer. The insulating coating layer may be made to at least partially overlap a boundary formed at an interface of the Ta₂O₅ layer and the Ta layer. The pacemaker can be an implantable cardiac pacemaker, and further be a leadless implantable cardiac pacemaker. Further, the capacitive electrode can be a capacitive return electrode, and the second electrode may be a metal-based stimulating electrode.

In another exemplary embodiment, a leadless pacemaker may include a pacemaker case having an anchoring area and a distal area, a capacitive electrode located within the distal area, the capacitive electrode having a hitch, a second electrode located in the anchoring area, and an anti-parallel Schottky junction formed by a semiconductor die (preferably a n-type silicon die) bonded to a portion of the hitch and in electrical connection with the capacitive electrode. Preferably the capacitive electrode can be a capacitive return electrode, and the second electrode may be a stimulating electrode. The anti-parallel Schottky junction can provide oxide reverse voltage protection for the pacemaker system by accommodating polarizable characteristics of the capacitive electrode. In some embodiments, to build a leadless pacemaker, a metal layer can be deposited on an outer surface of the pacemaker case, wherein a reserved area of the hitch is not covered with the metal layer. Further, an oxide layer may be deposited on at least a portion of the distal area to define the capacitive electrode. Further, a conductive layer can be applied to at least a portion of the distal area, at least to the oxide layer. The conductive layer can serve as a cathode of the capacitive electrode. In some embodiments, an interface at the conductive layer and the semiconductor die can behave as a Schottky junction. The pacemaker case, excluding the distal area, can be electrically isolated by an insulation coating layer. Additionally or alternatively, in some embodiments, the pacemaker case, excluding the distal area, is electrically isolated from the rest of the pacemaker case. Further, a portion of the pacemaker case having the insulating coating layer can be made to exhibit a capacitance that is less than the capacitance of the capacitive electrode.

In at least one embodiment the pacemaker can include: a pacemaker case with an electrically conductive area; a semiconductor die with an ohmic connection to the conductive area of the pacemaker case; a metal layer deposited on at least a portion of the pacemaker case; an oxide layer deposited or developed on at least a portion of the metal layer deposited; an insulating layer electrically isolating a portion of the pacemaker case having the metal layer and the oxide layer; and, a conductive layer deposited on at least a portion of the oxide layer, the insulating coating layer, and the semiconductor die; wherein a region comprising the metal layer, the oxide layer, and the conductive layer and the pacemaker case, and the region comprising the semiconductor die and the conductive layer define a capacitive electrode of the pacemaker. The pacemaker can be an implantable cardiac pacemaker, and further be a leadless implantable cardiac pacemaker. Preferably the semiconductor die may be a n-type silicon. In some embodiments, the conductive layer and the semiconductor die form a Schottky junction that prevents damage to at least the oxide layer when the pacemaker is subjected to external defibrillation pulses. In some embodiments, a capacitance of the capacitive electrode is greater than a capacitance of the insulating coating layer portion of the pacemaker case. In some embodiments, the conductive layer serves as a cathode of the capacitive electrode. In some embodiments, the oxide layer is a Ta₂O₅ layer. In some embodiments, the pacemaker further includes a battery can containing an assemblage of battery chemistry and battery components, wherein the anode of the capacitive electrode is connected to the negative terminal of the battery can. In some embodiments, at least a portion of the battery can serves as the pacemaker case.

In at least one embodiment, the pacemaker can include: a pacemaker case; a capacitive electrode that functions as a return electrode during pacing, wherein the capacitive electrode is formed of: a tantalum ("Ta") layer deposited on at least a portion of the pacemaker case; a tantalum pentoxide ("Ta₂O₅") layer deposited on at least a portion of the Ta layer; an insulating coating layer applied to the Ta layer and/or at least a portion of the Ta₂O₅ layer; and, a conductive layer deposited on at least a portion of the Ta layer, the Ta₂O₅ layer, and/or the insulating coating layer; wherein a Schottky junction is formed on at least a portion of the pacemaker case and is in electrical connection with the return electrode. In some embodiments, the Schottky junction provides oxide reverse voltage protection to the leadless pacemaker when the pacemaker is subjected to external defibrillation pulses. In some embodiments, the Ta₂O₅ layer is structured to have a porous outer layer and a dense inner layer. In some embodiments, the insulating coating layer at least partially overlaps a boundary formed at an interface of the Ta₂O₅ layer and the Ta layer. The pacemaker can be an implantable cardiac pacemaker, and further be a leadless implantable cardiac pacemaker.

In at least one embodiment, the leadless pacemaker can include: a pacemaker case having an anchoring area and a distal area with a hitch; a capacitive electrode located within the distal area, the capacitive electrode built within the hitch; a second electrode located in the anchoring area; and an anti-parallel Schottky junction with the capacitive electrode formed by semiconductor die (preferably a n-type silicon die) bonded to a portion of the hitch. Preferably the capacitive electrode may be a capacitive return electrode, and the second electrode may be a stimulating electrode. In some embodiments, the anti-parallel Schottky junction provides oxide reverse voltage protection for the leadless pacemaker by accommodating polarizable characteristics of the capacitive return electrode, for example when the pacemaker is subjected to external defibrillation pulses. In some embodiments, the leadless pacemaker further includes a metal layer deposited on an outer surface of the pacemaker case, wherein a reserved area of the hitch is not covered with the metal layer. In some alternative or additional embodiments, the leadless pacemaker further includes a metal layer deposited on an outer surface of the pacemaker case, wherein the reserved area of the hitch is utilized to bond the semiconductor die. These metal layers of both embodiments may be tantalum ("Ta") layers. In some embodiments, the leadless pacemaker further includes an oxide layer deposited on at least a portion of the distal area, preferably a portion of the deposited metal to define the capacitive electrode. The oxide layer may be a tantalum pentoxide ("Ta₂O₅") layer, preferably structured to have a porous outer layer and a dense inner layer. In some embodiments, the leadless pacemaker further includes a conductive layer applied to at least a portion of the distal area, preferably at least a portion of the deposited oxide layer. In some embodiments, the conductive layer serves as a cathode of the capacitive electrode. In some embodiments, an interface at the conductive layer and the semiconductor die behaves as a Schottky junction. In some embodiments, the pacemaker case, excluding the distal area, is electrically isolated by an insulation coating layer. Additionally or alternatively, in some embodiments, the pacemaker case, excluding the distal area, is electrically isolated from the rest of the pacemaker case. In some embodiments, a portion of the pacemaker case having the insulating coating layer exhibits a capacitance that is less than a capacitance of the return capacitive electrode. In some embodiments, the insulating coating layer at least partially overlaps a boundary formed at an interface of the oxide layer and the metal layer. In some embodiments, the pacemaker further includes a battery can containing an assemblage of battery chemistry and battery components, wherein the anode of the capacitive electrode is connected to the negative terminal of the battery can. In some embodiments, at least a portion of the battery can serves as the pacemaker case.

Further features, aspects, objects, advantages, and possible applications of the present invention will become apparent from a study of the exemplary embodiments and examples described below, in combination with the Figures, and the appended claims.

The above and other objects, aspects, features, advantages and possible applications of the present invention will be more apparent from the following more particular description thereof, presented in conjunction with the following drawings, in which:
- FIG. 1: is a prior art electrode that may be used with known pacemaker systems.
- FIG. 2: is a prior art implantable stimulator.
- FIG. 3: is an exemplary leadless pacemaker system, according to a preferred embodiment of the present invention, utilizing a capacitive electrode as a return electrode.
- FIGS. 4A-4B: show an exemplary leadless pacemaker system, according to a preferred embodiment of the present invention, with a battery can serving as part of a pacemaker case, and an exemplary arrangement of a plurality of layers that may be used to define the capacitive electrode, respectively.
- FIG. 5: shows an exemplary electrical schematic that can be used to represent a pacing front-end of an exemplary leadless pacemaker system according to a preferred embodiment of the present invention.
- FIG. 6: shows voltages excursions of the equivalent electrode-tissue capacitances for pacing amplitudes delivered through the pacing front-end represented by the exemplary electrical schematic of FIG. 5.
- FIG. 7: shows a voltage difference profile that may be experienced by an exemplary pacemaker system during an external defibrillation pulse.
- FIG. 8: shows an exemplary leadless pacemaker system with an n-type silicon ("Si") die bonded to a portion of a hitch according to a preferred embodiment of the present invention.
- FIGS. 9A-9C: show exemplary process steps that may be used to form a Schottky junction with the n-type Si die bonded arrangement of FIG. 8.
- FIG. 10: shows an exemplary electrical schematic that can be used to represent a pacing front-end of an exemplary leadless pacemaker system with an embedded anti-parallel Schottky junction within an assembly of the capacitive return electrode according to a preferred embodiment of the present invention.

The following description is of an embodiment presently contemplated for carrying out the present invention. This description is not to be taken in a limiting sense, but is made merely for the purpose of describing the general principles and features of the present invention. The scope of the present invention should be determined with reference to the claims.

The pacemaker system can include a pacemaker case and two electrodes, which may include a pacing electrode (*i.e.,* stimulating electrode) and a return electrode. Further embodiments can include each of the return electrode and the stimulating electrode capable of being associated with a pacemaker case. The return electrode can be a capacitive return electrode, which may be based on a dielectric. The capacitive return electrode can include an oxide layer, which may be a tantalum pentoxide ("Ta₂O₅"), an amorphous Ta₂O₅ layer, or other oxide layer. In some embodiments, the battery can may serve as the pacemaker case. Thus, the capacitive return electrode can be a Ta₂O₅ based capacitive return electrode built in and/or built on a portion of a pacemaker case and/or a battery can to form a leadless pacemaker system. The pacing electrode can be a noble-metal electrode, which may be fractal iridium ("fractal lr") for example. In at least one embodiment, the pacemaker case and/or the battery can may be constructed of titanium ("Ti").

An n-type silicon ("Si") die can be bonded to the pacemaker case or other portion of the pacemaker system, forming an ohmic contact (*e.g*., allowing for free current flow in both directions without rectification of any alternating current). Further, a metal layer can be deposited over at least a portion of the leadless pacemaker system. This may include a layer of tantalum ("Ta"). Some embodiments can further include growing the oxide layer on at least a portion of the leadless pacemaker system. This may include a growing the oxide layer on a selective area of the leadless pacemaker case, except for a portion of the unattached face of the n-type Silicon die ("n-type Si die").

The metal layer and/or the oxide layer may also be partially electrically isolated by an application of an insulating coating. The insulating coating layer can be a coating of parylene, for example. Such a configuration may permit the underlying metal layer to rapidly oxidize should the insulating coating layer become compromised.

A conductive layer may then be deposited on at least a portion of the oxide layer, the insulating coating layer, and an n-type Si die. The conductive layer may be exposed to blood and/or tissue during implantation, and thus it is envisioned for the conductive layer to be a biocompatible material. For example, the conductive layer can be poly(3,4-ethylenedioxythiophene):poly(styrenesulfonate) ("PEDOT:PSS"), a noble metal, etc. The conductive layer can be made to further act as a cathode for completing the capacitive return electrode, thereby forming a Schottky junction with the n-type Si die. This Schottky junction can be utilized for its rectifying characteristics, and thus limit current flow in one direction. In some embodiments, protection from external defibrillation pulses can be achieved via the Schottky junction.

Further, with any of the embodiments discussed herein, an equivalent capacitance of the capacitive return electrode can made to be greater than an equivalent capacitance of the insulating coating layer portion of the leadless pacemaker case.

As by way of a non-limiting example, a leadless pacemaker system can be generated with a Ta₂O₅ capacitive return electrode built on a battery can, wherein the battery can operates as the pacemaker case. Protection to the capacitive return electrode can be implemented by an n-type Si die that is bonded to the Ti pacemaker case (making an ohmic contact). An amorphous Ta₂O₅ layer can be grown on a selective area of the Ti leadless pacemaker case, except for a portion of the top of the n-type Si die. A conductive layer can finally be deposited over the Ta₂O₅ layer as the cathode completing the capacitive electrode and making a Schottky junction with the n-type Si. The rest of the Ti leadless pacemaker case can have a Ta layer deposited over it and be electrically isolated with a coating of parylene.

Turning now to the Figures, several specific embodiments are described in more detail. The various embodiments and features described in the depicted embodiments may be used either individually or in any appropriate combination, as the disclosure is not limited to any specific embodiment descripted herein.

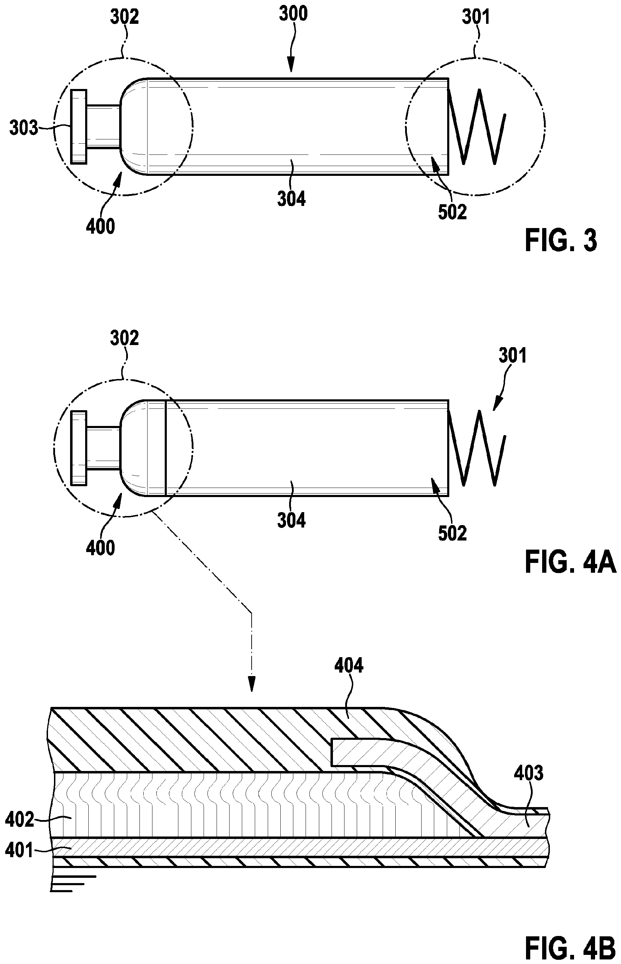

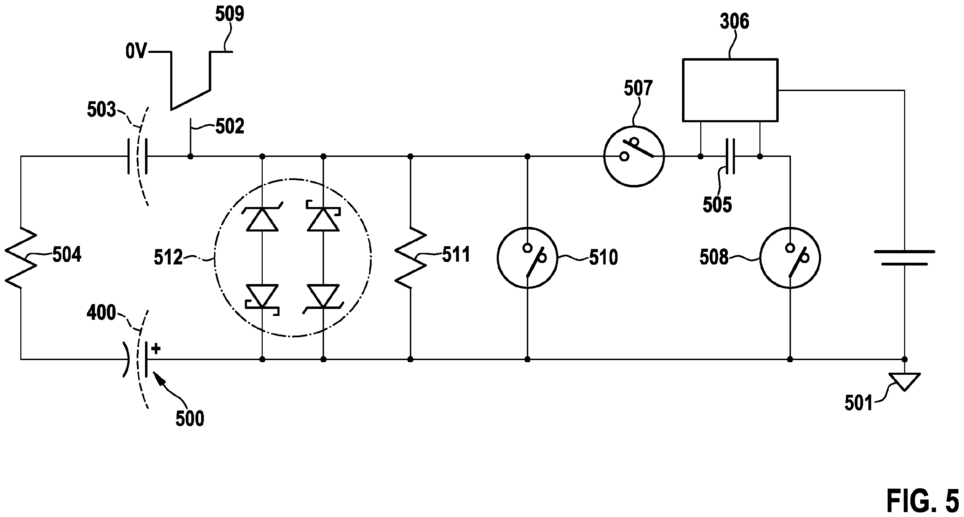

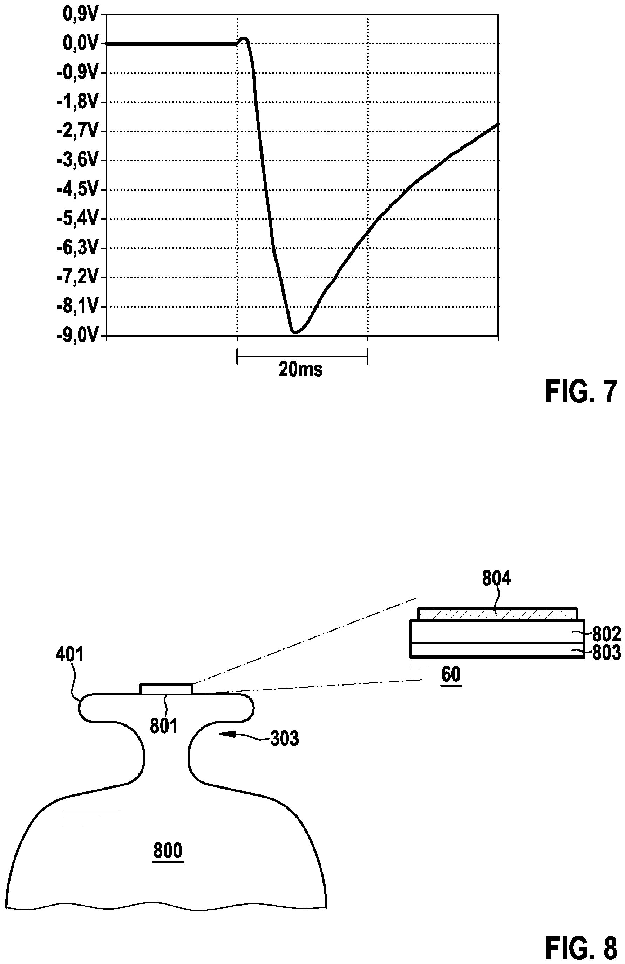

As shown in FIG. 3, the leadless pacemaker system 300 can include a pacemaker case 304 and two electrodes, which may be a pacing electrode (*i.e.,* stimulating electrode) 502 located in anchoring area 301 and a capacitive return electrode 400 located in distal area 302. The capacitive return electrode 400 may include the hitch 303 as part of an active portion of the capacitive return electrode 400. The hitch 303 may facilitate implantation and explantation of the leadless pacemaker system 300. At least a portion of the pacemaker case 304, excluding the distal area 302, can be electrically isolated by application of an insulating coating layer 403, *e.g*., parylene coating, (*see* FIG. 4B). This configuration can enable the leadless pacemaker system 300 to perform similar functions related to a traditional bipolar pacing channel. As noted above, at least a portion of the battery can 800 (*see* FIG. 8) may serve as the leadless pacemaker case 304. The battery can 800 may be made of titanium (Ti) and connected to the battery negative terminal 501 (*see* FIG. 5). Further, an anode 500 (*see* FIG. 5) of the capacitive return electrode 400 may be connected to such negative terminal 501 of the battery can 800 during implantation. This connection to the negative terminal 501 can be permanent.

Referring now to FIGS. 4A-4B, in further embodiments, the capacitive return electrode 400 can include both the hitch 303 and a distal top section of the leadless pacemaker case 304 (e.g. battery can 800). The metal layer 401 can be deposited on at least a portion of the leadless pacemaker case 304 (e.g. battery can 800). For example, the metal layer 401 can deposited over an outer surface of the leadless pacemaker case 304, which may include an entire outer surface of the leadless pacemaker case 304. The deposition of the metal layer 401 can be achieved via electrochemical deposition, reactive physical vapor deposition ("PVD"), and/or another suitable deposition process. In case a battery can 800 implements the leadless pacemaker case 304, it is envisioned for the deposition of the metal layer 401 to be completed before the assemblage of the battery chemistry and battery components for the battery can 800 is performed. The oxide layer 402 can then be grown on at least a portion of the distal area 302. The oxide can define the capacitive electrode. The oxide layer 402 can be formed by anodic oxidation, simple hydrolysis-condensation process at room temperature, etc. The resultant oxide layer 402 can be structured as a porous outer layer, shown as flaky lines, and a dense inner layer, shown as straight lines (*see* FIG. 4B).

A native metal oxide may be formed over the metal layer 401 during manufacturing. For example, if the metal layer 401 is Ta, the native metal oxide can be native Ta oxide. This native metal oxide layer, located at the rest of the leadless pacemaker case 304, may be stripped off. After stripping the native metal oxide layer, the insulating coating layer 403, *e.g.*, a parylene coating, can be deposited onto at least a portion of the leadless pacemaker case 304, which may include at least partially overlapping a boundary formed at an interface between the oxide layer 402 (e.g. Ta₂O₅) and the underlying metal layer 401. Although it may be unlikely, if the insulating coating layer 403 develops a short-through (*e.g.,* a scratch due to handling), the underlying metal layer 401 would be exposed, enabling the metal layer 401 to easily develop an oxide and thus prevent direct current flow. The portions of the leadless pacemaker case 304 having the insulating coating layer 403 covering the metal layer 401 and/or the oxide layer 402 can be made to exhibit an equivalent capacitance that is less than a capacitance of the return capacitive electrode 400.

Within this disclosure, the distal area 302 and the return capacitive electrode 400 are collectively referred to as the return electrode area. The conductive layer 404, preferably be poly(3,4-ethylenedioxythiophene):poly(styrenesulfonate) (also known as "PEDOT:PSS"), a biocompatible conductive polymer, can then be deposited over at least a portion of the return electrode area, which may be done to provide protection for the oxide layer 402. This can include depositing the conductive layer 404 on an entire outer surface of the return electrode area. The conductive layer 404 can be deposited via a galvanic process, chemical vapor deposition ("CVD") process, or other similar process. In some embodiments, applying a PEDOT:PSS conductive layer 404 with at least a Ta metal layer 401 may result in a final build-up, generating a pacemaker system 300 with the capacitive return electrode 400 acting as an equivalent Ta capacitor, a PEDOT:PSS cathode, and a Ta anode 500 (*see* FIG. 5) connected to a negative terminal 501 (*see* FIG. 5) of the battery can 800 (*see* FIG. 8). This connection to the negative terminal 501 can be permanent. The PEDOT:PSS cathode can further be used to facilitate contact with blood of a patient for current return during pacing operations of the leadless pacemaker system 300. Further, the leadless pacemaker system 300 can be configured so that an equivalent capacitor breakdown voltage is greater than 20 volts ("V") and for its capacitance to be in the order of, or approximately, 100 micro-Farads ("µF"). With such a configuration, the capacitive return electrode 400 can behave as a pure capacitor with a reverse voltage of up to (-1.0) V, according to cyclic voltammetry measurements. Thereafter, the battery chemistry and components can be assembled within the battery can 800, along with the built-in capacitive return electrode 400.

The stimulating electrode 502 (*see* FIG. 5) may be made to exhibit an equivalent Helmholtz capacitance in tissue of approximately 20 µF. This may result in an equivalent output pacing capacitance of approximately 16.7 µF, considering the in series 100 µF of the capacitive return electrode 400 during pacing.

FIG. 5 shows an exemplary electrical schematic that can be used to represent a pacing front-end of an exemplary leadless pacemaker system 300. As noted above, the capacitive return electrode 400 can have its anode 500 permanently tied to the battery negative terminal 501 (*e.g.,* system ground). An interface between the stimulating electrode 502 and tissue can be represented by non-polarizable capacitor 503, and a tissue resistance to pacing can be modeled by resistor 504. To deliver a pacing pulse, capacitor 505 can be precharged to a programmed pacing voltage by a voltage step-up/down circuit represented by block 506 (*e.g.* a charge pump). When a pacemaker state machine indicates that it is time to deliver a pace, electrical connections to block 506 can be floated and analog switches 507, 508 can be closed by internal control logic. This may force the pacing electrode 502 below ground 501 (*i.e.,* generate a negative voltage), allowing current to flow from terminal 500 through tissue resistance 504, resulting in a cathodic stimulation pulse 509 at the stimulating electrode 502. An auto-short can be performed following an end of such pulse 509 by opening analog switches 507, 508 and closing analog switch 510 for tens of miliseconds, which may allow discharging the stored capacitances of the tissue-electrodes (*e.g*., return electrode 400 and equivalent capacitance 503 of pacing electrode 502 and tissue).

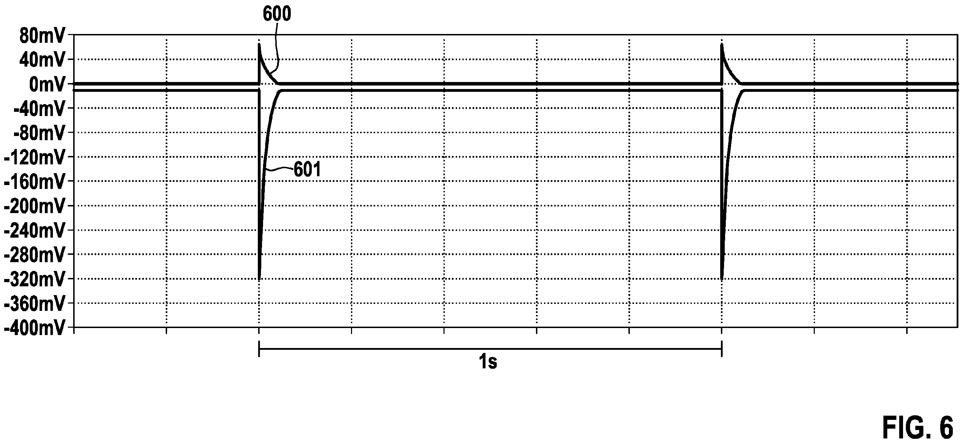

FIG. 6 shows voltages excursions of the stored capacitances described above for a large programmed pacing amplitude (*e.g*., 6.0 V) applied to the exemplary electrical schematic of FIG. 5. Trace 600 shows a voltage difference exhibited between terminal 500 and electrolyte within a vicinity of the capacitive return electrode 400 (which may include the return electrode area), which swings positively up to 65 mV. Trace 601, on the other hand, is a voltage difference exhibited between the pacing electrode 502 and electrolyte within its vicinity (i.e. charge across non-polarizable capacitor 503 in FIG. 5). Trace 601 swings to (-320 mV), which is within the safe operation range ("water window") for the noble metal electrode 502.

Although leadless pacemakers are not directly addressed by the present International Organization Standard ("ISO") 14708-2 standard (or equivalent), the electronic module of the disclosed leadless pacemaker system 300 is able to withstand without damage when a patient, that has been implanted with the leadless pacemaker system 300, is provided external defibrillation support. For example, in an effort to estimate a dipole that may exist between the electrodes 400, 502 of the leadless pacemaker 300 in the presence of an external defibrillation shock, the inventors have considered that the electronic field at the right ventricle ("RV") of a human heart to be less than 20.0 V/cm when a 300-Joule ("J") external defibrillation shock is applied to the patient. *See* Rosborough et al., "A Percutaneous Catheter-Based System for the Measurement of Potential Gradients Applicable to the Study of Transthoracic Defibrillation, Pacing and Clinical Electrophysiology", 30(2), pp. 166-174, February 2007. Extrapolating linearly in voltage, a 400-J maximum external shock, which may be possible with state-of-the art equipment, is expected to create approximately 23.1 V/cm at the RV. With a distance between electrodes 400, 502 in the leadless pacemaker system 300 being approximately 3.0 centimeters, the pacemaker system 300 may experience up to ±70 V between its terminals during external defibrillation with a 400-J pulse. Under such scenario, an internal junction-based protection element 512 will fully turn on and a voltage difference between terminal 500 and the electrolyte in the vicinity of the capacitive return electrode 400 will go negative several volts, as shown in FIG. 7. This reverse voltage may actually persist for several seconds because the bleeding-off resistor 511 for passive discharge can be large (*e.g,* hundreds of kΩ).

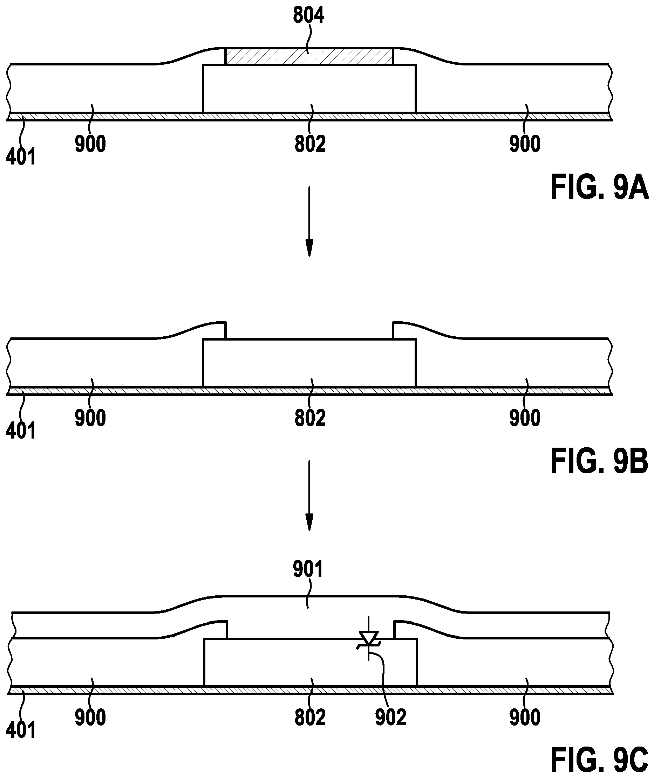

Referring now to FIG. 8, although Ta capacitors have been shown to exhibit the capability for oxide self-healing after being subject to transitory reverse voltages, the profile shown in FIG. 7 may damage the oxide layer 402 of the capacitive return electrode 400 beyond selfhealing, allowing for DC current leakage to flow. Thus, in another preferred embodiment, a Schottky junction 902 (*see* FIG. 9C) can be built into the capacitive return electrode 400 assembly to further protect it and/or the leadless pacemaker system 300 from external defibrillation pulses. With this embodiment, just as with the embodiments described above, the leadless pacemaker case 304 and/or battery can 800 may have a metal layer 401 deposited on at least a portion thereof before the battery chemistry and components are assembled for the battery can 800. In some embodiments, metal layer 401 can be deposited on an entire outer surface of the battery can 800, except for a reserved area 801. In further embodiments, the reserved area 801 may be a small area on the hitch 303, which can include a flat portion of the hitch 303. The deposition of the metal layer 401 can be achieved via electrochemical deposition, reactive physical vapor deposition ("PVD"), and/or another suitable deposition process.

Using a suitable die-bonding process, at least one n-type Si die 802 may be bonded to the reserved area 801, making ohmic contact via an n+ diffusion layer 803 to the battery can 800. It is noted that any die-bonding process typically employed for die packaging to a metal substrate may be used to form the die-bonding, which may include thermocompression. The n-type Si die 802 can be fabricated via wafer processing and dicing technology, each n-type Si die 802 being structured with an n+ diffusion layer 803 on one side and at least a partial passivation layer 804 on the opposite side (*e.g.,* silicon nitride (Si₃N₄)).

Referring to FIGS. 9A-9C, an oxide layer 900 can then be deposited in an area that defines the capacitive return electrode 400, resulting in the structure shown in the cross-sectional view of FIG. 9A. The oxide layer 900 can be deposited electrochemically, for example. Although not shown, silicon dioxide ("SiO₂") may be grown on at least one side of the n-type Si die 802 without affecting the performance of the capacitive return electrode 400. The passivation layer 804 can be utilized to function as a masking layer for the oxidation of the metal layer 401. The passivation layer 804 can then be etched, exposing the n-type Si die 802, as shown in FIG. 9B. This can be achieved by using a wet etching process. It is noted that different masking mechanisms may be utilized, depending on the deposition selected. The conductive layer 901 may then be deposited on at least a portion of the pacemaker system 300 to complete the capacitive return electrode 400, as shown FIG. 9C. In such a configuration, the conductive layer/die interface (*e.g*., the PEDOT:PSS/n-type-Si interface) can behave as a Schottky junction 902. A remaining portion of the battery can 800 that is not the capacitive return electrode 400 and/or the return electrode area may be stripped of native metal oxide, in a similar manner as described above. After stripping, the stripped area(s) may then be coated with the insulating layer 403, in a similar manner as described above. Thereafter, the battery chemistry and components can be assembled within the battery can 800, along with the built-in capacitive return electrode 400 and the anti-parallel Schottky junction 902.

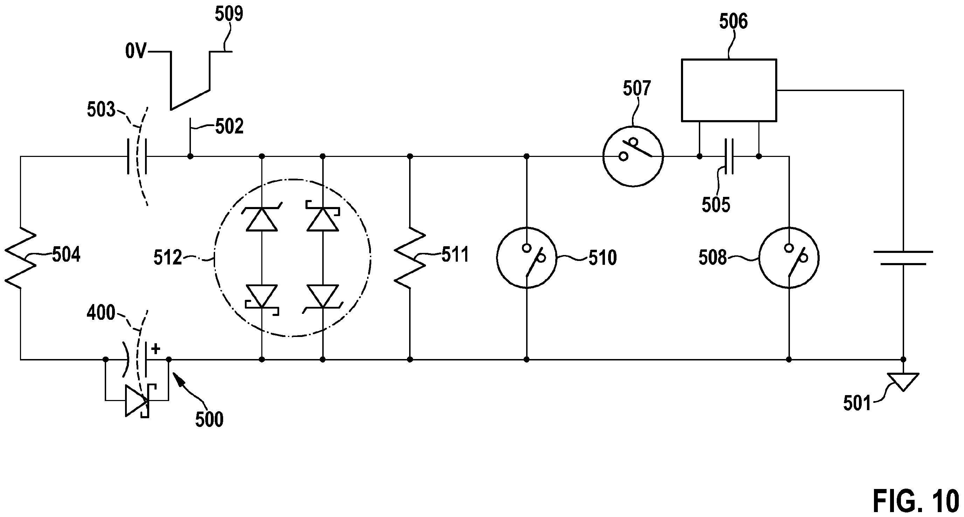

FIG. 10 shows an exemplary electrical schematic that can be used to represent the pacing front-end of the leadless pacemaker 300 with an embedded anti-parallel Schottky junction 902 in an assembly of the capacitive return electrode 400. Using the exemplary electrical schematic of FIG. 10, a voltage difference between terminal 500 and the electrolyte in the vicinity of the capacitive return electrode 400 (which may include the return electrode area) swings to (-300 mV) in the presence of an external defibrillation pulse, which is within the operating range of the capacitive return electrode 400, as discussed above.

In at least one embodiment, a capacitive return electrode 400 of the leadless pacemaker system 300 is protected from external defibrillation pulses without utilizing a feedthrough. Thus, the leadless pacemaker system 300 can be structured such that only a pacing electrode 502 is placed through a feedthrough.

The present invention avoids the need for the traditional large discrete-component direct current (DC) blocking capacitor placed in series with the pacemaker pacing circuit while still complying with DC leakage current requirements. Unlike prior art, the present invention discloses a return capacitive electrode without the need of voltage pre-biasing and with built-in protection for its oxide against external defibrillation pulses.

From the strategic point of view, the present invention allows implementing a leadless pacemaker with built-in series capacitor, which has not been proposed in prior art.

## Claims

1. A leadless pacemaker, comprising:
a pacemaker case having an anchoring area and a distal area with a hitch;
a capacitive electrode located within the distal area, the capacitive electrode built within the hitch;
a second electrode located in the anchoring area; and
an anti-parallel Schottky junction with the capacitive electrode formed by semiconductor die bonded to a portion of the hitch.

2. The leadless pacemaker recited in claim 1, wherein the anti-parallel Schottky junction provides oxide reverse voltage protection for the leadless pacemaker by accommodating polarizable characteristics of the capacitive electrode.

3. The leadless pacemaker recited in claim 1, further comprising a conductive layer applied to at least a portion of the distal area.

4. The leadless pacemaker recited in claim 3, wherein the conductive layer serves as a cathode of the capacitive electrode.

5. The leadless pacemaker recited in one of the preceding claims, wherein an interface at the conductive layer and the semiconductor die behaves as a Schottky junction.

6. The leadless pacemaker recited in claim 1, further comprising a metal layer deposited on an outer surface of the pacemaker case, wherein a reserved area of the hitch is not covered with the metal layer.

7. The leadless pacemaker recited in claim 6, wherein the metal layer is a tantalum ("Ta") layer.

8. The leadless pacemaker recited in claim 1, further comprising an oxide layer deposited on at least a portion of the distal area.

9. The leadless pacemaker recited in claim 8, wherein the oxide layer is a Ta₂O₅ layer, preferably the Ta₂O₅ layer is structured to have a porous outer layer and a dense inner layer.

10. The leadless pacemaker recited in claim 1, wherein the pacemaker case, excluding the distal area, is electrically isolated by an insulation coating layer.

11. The leadless pacemaker recited in claim 10, wherein a portion of the pacemaker case having the insulating coating layer exhibits a capacitance that is less than a capacitance of the capacitive electrode.

12. The pacemaker recited in one of the claims 6 to 11, wherein the insulating coating layer at least partially overlaps a boundary formed at an interface of the oxide layer and the metal layer.

13. The leadless pacemaker recited in claim 1, further comprising a battery can containing an assemblage of battery chemistry and battery components, wherein an anode of the capacitive electrode is connected to a negative terminal of the battery can.

14. The leadless pacemaker recited in claim 13, wherein at least a portion of the battery can serves as the pacemaker case.
